# EUROPÄISCHE PATENTSCHRIFT

(11) **EP 3 319 546 B1**
(45) Veröffentlichungstag und Bekanntmachung des Hinweises auf die Patenterteilung: **21.10.2020**
(21) Anmeldenummer: 16747450.1
(22) Anmeldetag: 06.07.2016
(51) Int. Cl.: A61C 5/70, A61C 8/00

(54) **HERSTELLUNGSVERFAHREN FÜR ZAHNERSATZTEIL UND VERBLENDSTRUKTUR**
PRODUCTION METHOD FOR TOOTH REPLACEMENT PART AND VENEER STRUCTURE
PROCÉDÉ DE FABRICATION D'UN ÉLÉMENT DE PROTHÈSE DENTAIRE ET STRUCTURE À INCRUSTATION VESTIBULAIRE

(30) Priorität: 06.07.2015 DE 102015212606
(43) Veröffentlichungstag der Anmeldung: 16.05.2018
(73) Patentinhaber: Sirona Dental Systems GmbH, 64625 Bensheim (DE)
(72) Erfinder: SCHNITZSPAN, Paul, 64342 Seeheim-Jugenheim (DE); JORDAN, Thorsten, 64319 Pfungstadt (DE); RÖSSLER, Friedemann, 64625 Bensheim (DE)
(74) Vertreter: Özer, Alpdeniz
(86) Internationale Anmeldenummer: PCT/EP2016/065898
(87) Internationale Veröffentlichungsnummer: WO 2017/005762

(56) Entgegenhaltungen:
- WO-A1-2012/163466
- WO-A1-2014/033323
- US-A1- 2011 065 065
- US-A1- 2015 044 635

## Beschreibung

### Technisches Gebiet

Die Erfindung betrifft ein Verfahren eines an einem Implantat zu befestigenden Zahnersatzteils ausgehend von einem 3D-Modell des Zahnersatzteils, wobei in dem 3D-Modell des Zahnersatzteils ein durchgehender Schraubenkanal für eine Fixierschraube mit einer zylindermantelförmigen Anfangsform automatisch und/oder manuell erzeugt wird, sowie ein entsprechende Verblendstruktur.

### Stand der Technik

Aus dem Stand der Technik sind unterschiedliche Arten zur Befestigung eines Zahnersatzteils an einem Implantat bekannt.

Aus der DE 692 15 106 T2 ist ein Verfahren zur Versorgung einer dentalen Fehlstelle bekannt, wobei ein übergroßer und zahnförmiger Implantatkopf an einem Implantat befestigt und mit einem relativ dünnen Überzug als äußere Zahnoberfläche versehen wird.

Die WO 2012/163466 A1 beschreibt ein aus einem Gerüst und einer Verblendstruktur aufgebautes Zahnersatzteil.

Die US 2003/0096214 A1 beschreibt ein Verfahren zur Herstellung von Zahnersatzteilen mit digitalen Verfahrensschritten.

Aus der WO 2012/052482 A1 ist ein Verfahren zur Bereitstellung eines patientenspezifischen Befestigungssystems für ein Zahnersatzteil bekannt, wobei ein Implantat mittels eines Abutments versorgt und ein Zahnersatzteil mit einer entsprechenden Ausnehmung auf das Abutment geklebt wird.

Entsprechende Versorgungen ist auch aus der US 2015/044635 A1 bekannt.

Nachteilig ist, ein Entfernen des Zahnersatzteils, z.B. um die Schraube zu Kontrollieren oder Nachzuziehen, nicht ohne Zerstören des Zahnersatzteils möglich ist.

Einen einfacheren Zugang zur Schraube kann dadurch erreicht werden, dass das Abutment sowie das Zahnersatzteil einen durchgehenden Schraubenkanal für eine Fixierschraube aufweisen, der nach dem Festschrauben mittels einer Komposit-Füllung geschlossen wird. Durch entfernen des Komposits ist die Schraube leicht zugänglich. Nachteilig ist jedoch, dass Teile des Abutments und der Schraube durch die Komposit-Füllung häufig durchscheinen und sich dadurch der Schraubenkanal gegenüber dem Zahnersatzteil dunkel abhebt.

Aus der US 2011/065065 A1 ist bekannt, einen Schraubenkanal eines Zahnersatzteils mit einem Zapfen zu verschließen, wobei Zapfen und Zahnersatzeil möglichst aus demselben Material gefertigt sind.

Gemäß der US 2015/0079542 A1 kann dieser Effekt abgeschwächt werden, indem das Abutment zweiteilig aus einem Kern und einem Mantel ausgebildet wird und der Mantel farblich dem Zahnersatzteil entspricht. Das Durchscheinen eines Schraubenkopfs einer Fixierschraube kann hierdurch jedoch auch nicht verhindert werden.

Aufgabe der Erfindung ist es, eine Alternatives Verfahren bzw. Zahnersatzteil zur Versorgung einer Fehlstelle bereitzustellen, um die vorgenannten Nachteile zu überwinden und den Stand der Technik weiterzubilden.

### Darstellung der Erfindung

Ein Gegenstand der Erfindung ist ein Verfahren zur Herstellung eines an einem Implantat zu befestigenden Zahnersatzteils ausgehend von einem 3D-Modell des Zahnersatzteils, wobei in dem 3D-Modell des Zahnersatzteils ein durchgehender Schraubenkanal für eine Fixierschraube mit einer zylindermantelförmigen Anfangsform, einem ersten implantatseitigen Ende und einem zweiten okklusal- oder inzisalseitigen Ende automatisch und/oder manuell erzeugt wird. Anschließend wird eine Querschnittsfläche des Schraubenkanals zum zweiten Ende des Schraubenkanals hin gleichbleibend oder zunehmend ausgestaltet und als Endform des Schraubenkanals gespeichert. Dann wird ein 3D-Modell eines Inlays mit einer Mantelfläche, einer Oberfläche und einer Unterseite erzeugt, wobei als Oberseite ein durch das zweite Ende der Endform des Schraubenkanals ausgeschnittener Teil der Oberfläche des 3D-Modells des Zahnersatzteils verwendet wird und die Mantelfläche als Negativform eines an das zweite Ende angrenzenden Teils der Endform des Schraubenkanals ausgebildet wird.

Es sei angemerkt, dass das Zahnersatzteil jede bekannte Verblendstruktur zur Versorgung einer dentalen Fehlstelle sein kann, die mittels einer Fixierschraube befestigt wird. Insbesondere kann das Zahnersatzteil eine Krone oder eine Brücke sein. Die Verblendstruktur kann einstückig oder auch mehrstückig ausgebildet sein. Die Verblendstruktur kann beispielsweise aus einem Abutment und einer Krone bestehen, wobei die Krone den mittels Inlays zu füllenden Schraubenkanal enthält.

Ferner sei angemerkt, dass bei gleichbleibender Querschnittfläche des Schraubenkanals, die ursprüngliche Form als Vorlage für das Inlay verwendet wird. Eine Zunahme kann eine beliebige Form haben, d.h., die Zunahme kann stufenweise und/oder kontinuierlich ausgestaltet sein und auch nur einzelne Bereiche des Umfangs der Querschnittsfläche betreffen, so dass sich die Form der Querschnittfläche ändert. Wird die Querschnittsfläche zum Ende hin zunehmend ausgestaltet, so bedeutet dies lediglich, dass zumindest die Querschnittfläche des zweiten Endes einen größeren Flächeninhalt hat, als die Querschnittsfläche des ersten Endes.

Als Inlay wird ein zweites in eine entsprechende Ausnehmung der Verblendstruktur eingepasstes Bauteil bezeichnet. Dies kann insbesondere bei im Bereich der Backenzähne auch ein üblicherweise als Onlay bezeichnetes Bauteil sein, welches einen größeren Bereich der lingualen und/oder palatinalen Oberfläche des Zahnersatzteils einnimmt.

Durch die in Richtung der inzisalen, okklusalen oder oralen Oberfläche zunehmende Querschnittsfläche des Schraubenkanals kann das Inlay in diesen einfach eingesetzt und stoffschlüssig verbunden werden, ohne dass beispielsweise das Bindemittel, auf der inzisalen oder okklusalen Oberfläche erkennbar ist.

Zur Ausgestalten des Schraubenkanals können ein maximaler Durchmesser oder ein Umfang der Querschnittfläche auf der inzisalen, okklusalen oder oralen Oberfläche und/oder eine die Zunahme festlegende Steigung oder Kurve oder eine Länge, über die sich die Zunahme erstrecken soll, vorab festgelegt oder während der Durchführung des Verfahrens abgefragt werden.

Wahlmöglichkeiten eines Anwenders, z.B. durch Einzeichnen oder Markieren von Punkten oder Linien im 3D-Modell oder durch Festlegen von Parametern, können durch vorab festgelegte Grenzwerte oder Rahmenbedingungen beschränkt werden. Beispielsweise kann ein Mindestwert für die Steigung, ein Maximalwert für die Länge, über welche sich die Zunahme erstrecken darf, oder eine Mindestzunahme der Querschnittsfläche hinterlegt sein.

Das Füllen des Schraubenkanals mit einem Inlay stellt sicher, dass die farbliche Gestalt der inzisalen, okklusalen oder oralen Oberfläche einer finalen Verblendstruktur aus Zahnersatzteil und Inlay gleichmäßig ist und insbesondere der Bereich oberhalb des Schraubenkanals farblich der restlichen Oberfläche des Zahnersatzteils entspricht.

Ein Vorteil des erfindungsgemäßen Verfahrens ist, dass gleichzeitig eine einfache Zugänglichkeit der Fixierschraube durch entfernen des Inlays und ohne zerstören des restlichen Zahnersatzteils sichergestellt und eine möglichst einem natürlichen Zahn ähnelnde Oberfläche mit einer gleichmäßigen Färbung erreicht wird.

Vorteilhafterweise wird zur Ausgestaltung der Zunahme des Schraubenkanals auf der Oberfläche des 3D-Modells des Zahnersatzteils manuell und/oder automatisch eine die zylindrische Anfangsform des Schraubenkanals in Umfangsrichtung umschließende Linie erzeugt, automatisch und/oder manuell eine Steigung oder eine Länge der Zunahme festgelegt und die Endform des Schraubenkanals mit der festgelegten Steigung oder über die festgelegte Länge hinweg bis zu der Linie zunehmend ausgestaltet. Dies ermöglicht es einem Anwender auf einfache Weise die Zunahme der Querschnittsfläche des Schraubenkanals zu gestalten oder nachzuvollziehen.

Vorteilhafterweise wird die Mantelfläche des 3D-Modells des Inlays automatisch und/oder manuell zumindest teilweise in Richtung einer Längsachse des 3D-Modells des Inlays verschoben. So weist eine aus Zahnersatzteil und Inlay zusammengesetzte Verblendstruktur einen Spalt für ein Bindemittel, z.B. einen Kleber, auf.

Vorteilhafterweise wird die Querschnittsfläche des Schraubenkanals im Bereich der Zunahme zur Verdrehsicherung mit einer in Umfangsrichtung eindeutigen, nicht rotationssymmetrischen Form ausgestaltet. Eine Umfangslinie der Querschnittfläche des Schraubenkanals im Bereich des zweiten Endes kann beispielsweise eine von der Kreisform der zylindermantelförmigen Anfangsform abweichende Wölbung aufweisen. Mit eindeutig bzw. nicht rotationssymmetrisch wird eine Form bzw. Ausgestaltung der Querschnittsfläche bezeichnet, die sicherstellt, dass das Inlay beim Einsetzen in den Schraubenkanal hinsichtlich einer Rotation um eine Längsachse des Schraubenkanals richtig positioniert wird, so dass sich die Oberfläche des Zahnersatzteils und des Inlays zu einer möglichst zahnähnlichen Oberfläche ergänzen.

Die Erfindung betrifft weiterhin eine Verblendstruktur zur Versorgung einer dentalen Fehlstelle, bestehend aus einem Zahnersatzteil mit einem durchgehenden Schraubenkanal für eine Fixierschraube, der Schraubenkanal zumindest in einem an ein inzisales oder okklusales zweites Ende des Schraubenkanals angrenzenden Bereich zu dem inzisalen, okklusalen oder oralen Bereich hin eine gleichbleibende oder zunehmende Querschnittsfläche aufweist. Die Verblendstruktur umfasst ferner ein Inlay mit einer Oberseite, einer Unterseite und einer Mantelfläche, wobei die Mantelfläche einer Negativform eines an die inzisale, okklusale oder orale Oberfläche angrenzenden Teils des Schraubenkanals entspricht und die Oberseite eine Fortsetzung der inzisalen, okklusalen oder oralen Oberfläche im Bereich des Schraubenkanals darstellt.

Der Vorteil der erfindungsgemäßen Verblendstruktur ist die leichte Zugänglichkeit einer die Verblenstruktur am Implantat befestigenden Fixierschraube einerseits und die Sicherstellung einer einheitlichen Farbgebung bzw. ausreichend zahnähnlichen Gestalt der gesamten Oberfläche der Verblenstruktur andererseits.

Vorteilhafterweise weist der Schraubenkanal in dem an das zweite Ende angrenzenden Bereich eine in Umfangsrichtung eindeutige Form auf, die eine in Umfangrichtung eindeutige Orientierung zwischen dem Zahnersatzteil und dem Inlay sicherstellt.

### Kurzbeschreibung der Zeichnungen

Ausführungsbeispiele der Erfindung sind in der Zeichnung dargestellt. Es zeigt die
- Fig. 1: ein 3D-Modell eines Zahnersatzteils und die
- Fig. 2: ein Inlay zur Ergänzung des Zahnersatzteils.

### Ausführungsbeispiele

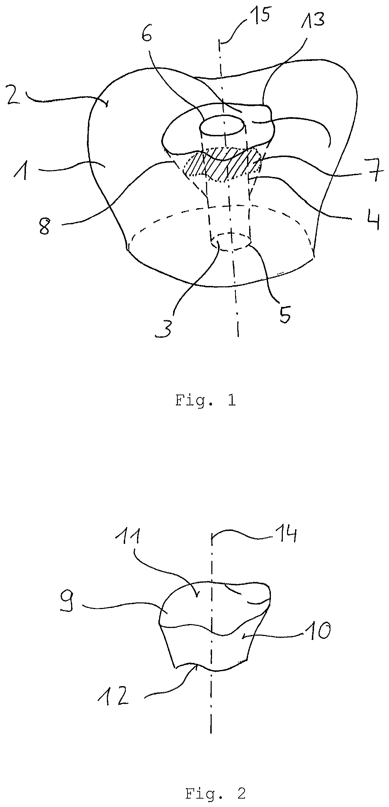

Die Fig. 1 zeigt eine schematische Darstellung eines 3D-Modells eines Zahnersatzteils 1 zur CAM-gestützten Herstellung eines an einem Implantat (nicht dargestellt) zu befestigenden Zahnersatzteils für einen BackenAzahn. Das 3D-Modell des Zahnersatzteils 1 weist eine Oberfläche 2 aus einer okklusalen Fläche, Seitenflächen und einer Unterseite auf, wobei insbesondere die okklusale Fläche sowie die Seitenflächen bereits die gewünschte Form bzw. Struktur einer finalen Verblendstruktur zum Versorgen einer dentalen Fehlstelle haben. Ein Schraubenkanal 3 für eine Fixierschraube (nicht dargestellt) durchdringt das 3D-Modell des Zahnersatzteils 1 von der Unterseite bis zur okklusalen Fläche.

Der Schraubenkanal 3 wird manuell und/oder automatisch erzeugt und weist eine zylindermantelförmige Anfangsform 4 mit einem ersten implantatseitigen Ende 5 und einem zweiten okklusalseitigen Ende 6 auf. Für die Erzeugung der Anfangsform 4 muss insbesondere die Implantatachse, des mit dem Zahnersatzteil zu versorgenden Implantats, und ein Durchmesser der Fixierschraube berücksichtigt werden.

Ausgehend von der Anfangsform 4 wird eine Endform 8 des Schraubenkanals 3 erzeugt, welche eine in Richtung des zweiten Endes 6, also in Richtung der okklusalen Oberfläche zunehmende Querschnittsfläche 5 aufweist. Hierfür wird auf der okklusalen Fläche des 3D-Modells des Zahnersatzteils 1 eine das zweite Ende 6 des Schraubenkanals vollständig umschließende Linie 13 automatisch und/oder manuell, z.B. mittels eines Eingabemittels, wie einer Computer-Maus, eingezeichnet. Die Linie kann kreisförmig sein, mit einem Durchmesser, der größer als der Durchmesser der zylindermantelförmigen Anfangsform 4 ist. Um eine in Drehrichtung um eine Längsachse 15 des Schraubenkanals 3 eindeutige Anschlussgeometrie bereitzustellen kann die Linie auch einen wellenförmigen und nicht rotationssymmetrischen Verlauf aufweisen, wie er in Fig. 1 skizziert ist.

Anschließend wird der Verlauf der Endform 8 des Schraubenkanals 3 erzeugt, indem die zylindermantelförmige Anfangsform zumindest im Bereich des zweiten Endes 6 bis zu der Linie 13 aufgeweitet wird, so dass die Linie 13 das zweite Ende 6 des Schraubenkanals 3 bildet. Hierfür kann vorab und/oder durch eine Eingabe eines Anwenders ein fester Steigungswert, ein Steigungsverlauf oder auch eine Länge, über welche sich das Aufweiten bzw. die Zunahme zu dem zweiten Ende 6 aus erstrecken soll, festgelegt werden. Der resultierende Schraubenkanal 3 mit einem zylindermantelförmigen Anfang im Bereich des ersten Endes 5 und einem beispielsweise kegelstumpfförmig zunehmenden Ende im Bereich des zweiten Endes 6 wird als Endform 8 abgespeichert.

Anschließend wird ausgehend von der Endform 8 des Schraubenkanals 3 ein 3D-Datensatz eines in das zweite Ende 6 des Schraubenkanals 3 hinein passenden Inlays 9 erzeugt, wie es in Fig. 2 schematisch dargestellt ist. Hierfür wird ein durch das zweite Ende 6 des Schraubenkanals 3 aus der ocklusalen Fläche des 3D-Dantensatzes des Zahnersatzteils 1 herausgeschnittener Teil der Oberfläche als eine Oberseite 11 verwendet. Eine Mantelfläche 10 des Inlays 9 wird als Negativform eines Teils der Endform 8 des Schraubenkanals 3 im Bereich des zweiten Endes 6 ausgebildet und durch eine das Inlay abschließende Unterseite 12 ergänzt.

Durch ein automatisches Erzeugen oder durch vorgegebene Randbedingungen bei manuellem Erzeugen der Endform 8 des Schraubenkanals 3 und der Erzeugung des Inlays 9 kann sichergestellt werden, dass das fertige Zahnersatzteil 1 zusammen mit dem Inlay 9, den gewünschten Anforderungen an Ästhetik, Stabilität und Langlebigkeit genügt. Beispielsweise kann durch das festlegen eines Minimalwerts für die Länge, über welche sich die Zunahme des Schraubenkanals 3 erstreckt, sichergestellt werden, dass das darin einzupassende Inlay eine ausreichende Höhe bzw. Dicke aufweist, um aus einem gewünschten Material sicher hergestellt zu werden und den farblichen bzw. ästhetischen Anforderungen zu entsprechen.

Um einen sicheren Halt des Inlays im Schraubenkanals 3 sicherzustellen kann, wie in Fig. 2 gestrichelt eingezeichnet, die Mantelfläche 10 in einem nächsten Verfahrensschritt zumindest teilweise in Richtung einer Längsachse 14 des Inlays 9 verschoben werden. So bildet sich nach einem Einsetzen des Inlays 9 in das Zahnersatzteil 1 ein Spalt für ein Bindemittel, z.B. einen Kleber, aus.

### Bezugszeichenliste

- 1: 3D-Modell des Zahnersatzteils
- 2: Oberfläche des 3D-Modells des Zahnersatzteils
- 3: Schraubenkanal
- 4: Anfangsform des Schraubenkanals
- 5: erstes Ende des Schraubenkanals
- 6: zweites Ende des Schraubenkanals
- 7: Querschnittsfläche des Schraubenkanals
- 8: Endform des Schraubenkanals
- 9: Inlay
- 10: Mantelfläche des Inlays
- 11: Oberseite des Inlays
- 12: Unterseite des Inlays
- 13: Linie
- 14: Längsachse des Inlays
- 15: Längsachse des Schraubenkanals

## Patentansprüche

1. Verfahren zur Herstellung eines an einem Implantat zu befestigenden Zahnersatzteils ausgehend von einem 3D-Modell des Zahnersatzteils (1) mit einer Oberfläche (2), wobei in dem 3D-Modell des Zahnersatzteils (1) ein durchgehender Schraubenkanal (3) für eine Fixierschraube mit einer zylindermantelförmigen Anfangsform (4), einem ersten implantatseitigen Ende (5) und einem zweiten okklusal- oder inzisalseitigen Ende (6) automatisch und/oder manuell erzeugt wird, wobei
- eine Querschnittsfläche (7) des Schraubenkanals (3) zum zweiten Ende (6) des Schraubenkanals (3) hin gleichbleibend oder zunehmend ausgestaltet und die daraus entstehende Form als Endform (8) des Schraubenkanals (3) gespeichert wird und
- wobei ein 3D-Modell eines Inlays (9) mit einer Mantelfläche (10), einer Oberseite (11) und einer Unterseite (12) erzeugt wird, wobei die Mantelfläche (10) als Negativform eines an das zweite Ende (6) angrenzenden Teils der Endform (8) des Schraubenkanals (3) ausgebildet wird, **dadurch gekennzeichnet, dass**
als Oberseite (11) ein durch das zweite Ende (6) der Endform (8) des Schraubenkanals (3) ausgeschnittener Teil der Oberfläche (2) des 3D-Modells des Zahnersatzteil (1) verwendet wird und die Mantelfläche (10) als Negativform eines an das zweite Ende (6) angrenzenden Teils der Endform (8) des Schraubenkanals (3) ausgebildet wird.

2. Verfahren nach Anspruch 1, **dadurch gekennzeichnet, dass** zur Ausgestaltung der Zunahme des Schraubenkanals (3) auf der Oberfläche (2) des 3D-Modells des Zahnersatzteils (1) manuell und/oder automatisch eine die zylindrische Anfangsform (4) des Schraubenkanals (3) in Umfangsrichtung umschließende Linie (13) erzeugt wird, dass automatisch und/oder manuell eine Steigung oder eine Länge der Zunahme festgelegt wird und dass die Endform (6) des Schraubenkanals (3) mit der festgelegten Steigung oder über die festgelegte Länge hinweg bis zu der Linie (13) zunehmend ausgestaltet wird.

3. Verfahren nach Anspruch 1 oder 2, **dadurch gekennzeichnet, dass** die Mantelfläche (10) des 3D-Modells des Inlays (9) automatisch und/oder manuell zumindest teilweise in Richtung einer Längsachse (14) des 3D-Modells des Inlays (9) verschoben wird.

4. Verfahren nach einem der Ansprüche 1 bis 3, **dadurch gekennzeichnet, dass** die Querschnittsfläche (7) des Schraubenkanals im Bereich der Zunahme zur Verdrehsicherung mit einer in Umfangsrichtung eindeutige, nicht rotationssymmetrische Form ausgestaltet wird.

5. Verblendstruktur zur Versorgung einer dentalen Fehlstelle, bestehend aus einem Zahnersatzteil (1) mit einem durchgehenden Schraubenkanal (3) für eine Fixierschraube sowie aus einem Inlay (9) mit einer Oberseite (11), einer Unterseite (12) und einer Mantelfläche (10), wobei der Schraubenkanal (3) zumindest in einem an ein inzisales oder okklusales zweites Ende (6) des Schraubenkanals (3) angrenzenden Bereich zu dem inzisalen, okklusalen oder oralen Bereich hin eine gleichbleibende oder zunehmende Querschnittsfläche (7) aufweist und wobei die Mantelfläche (10) einer Negativform eines an die inzisale, okklusale oder orale Oberfläche angrenzenden Teils des Schraubenkanals (3) entspricht **dadurch gekennzeichnet, dass** die Oberseite (11) eine Fortsetzung der inzisalen, okklusalen oder oralen Oberfläche im Bereich des Schraubenkanals (3) darstellt.

6. Verblendstruktur nach Anspruch 5, **dadurch gekennzeichnet, dass** der Schraubenkanal (3) in dem an das zweite Ende (6) angrenzenden Bereich eine in Umfangsrichtung eindeutige Form aufweist, die eine in Umfangrichtung eindeutige Orientierung zwischen dem Zahnersatzteil (1) und dem Inlay (9) sicherstellt.

## Claims

1. A method for producing a tooth replacement part to be attached to an implant on the basis of a 3D model of the tooth replacement part (1) having a surface (2), wherein in the 3D model of the tooth replacement part (1) a continuous screw channel (3) for a fixing screw having a cylindrical jacket-shaped initial shape (4), a first implant-side end (5) and a second occlusal-side or incisal-side end (6) is generated automatically and/or manually, wherein
- a cross-sectional area (7) of the screw channel (3) is designed to be constant or increasing towards the second end (6) of the screw channel (3) and the resulting shape is saved as the final shape (8) of the screw channel (3), and
- wherein a 3D model of an inlay (9) having a lateral surface (10), an upper side (11) and an underside (12) is produced, wherein the lateral surface (10) is a negative form of a part of the final shape (8) of the screw channel (3) adjacent to the second end (6), **characterised in that** a part of the surface (2) of the 3D model of the tooth replacement part (1) cut out by the second end (6) of the final shape (8) of the screw channel (3) is used as the upper side (11) and the lateral surface (10) is designed as a negative form of a part of the end form (8) of the screw channel (3) adjoining the second end (6).

2. The method according to claim 1, **characterised in that**, to design the increase of the screw channel (3) on the surface (2) of the 3D model of the tooth replacement part (1), a line (13) enclosing the cylindrical initial shape (4) of the screw channel (3) in the circumferential direction is manually and/or automatically generated, that a slope or a length of the increase is automatically and/or manually defined and that the final shape (6) of the screw channel (3) is designed to increase with the defined slope or over the defined length up to the line (13).

3. The method according to claim 1 or 2, **characterised in that** the lateral surface (10) of the 3D model of the inlay (9) is automatically and/or manually shifted at least partially in the direction of a longitudinal axis (14) of the 3D model of the inlay (9).

4. The method according to any one of claims 1 to 3, **characterised in that** the cross-sectional area (7) of the screw channel in the region of the increase is designed with a clear, non-rotationally symmetrical shape in the circumferential direction to prevent rotation.

5. A veneer structure for providing a dental defect, consisting of a tooth replacement part (1) having a continuous screw channel (3) for a fixing screw and an inlay (9) having an upper side (11), an underside (12) and a lateral surface (10), wherein the screw channel (3) has a constant or increasing cross-sectional area (7) at least in a region adjacent to an incisal or occlusal second end (6) of the screw channel (3) towards the incisal, occlusal or oral region and wherein the lateral surface (10) corresponds to a negative form of a part of the screw channel (3) adjoining the incisal, occlusal or oral surface, **characterised in that** the upper side (11) represents a continuation of the incisal, occlusal or oral surface in the region of the screw channel (3).

6. The veneer structure according to claim 5, **characterised in that** the screw channel (3) in the region adjacent to the second end (6) has a clear shape in the circumferential direction, which ensures clear orientation in the circumferential direction between the tooth replacement part (1) and the inlay (9).

## Revendications

1. Procédé de fabrication d'un élément de prothèse dentaire à fixer sur un implant, à partir d'un modèle 3D de l'élément de prothèse dentaire (1) avec une surface (2), dans lequel dans le modèle 3D de l'élément de prothèse dentaire (1) un canal de vis traversant (3) pour une vis de fixation avec une forme initiale d'enveloppe cylindrique (4), une première extrémité côté implant (5) et une seconde extrémité côté occlusal ou incisif (6) est généré automatiquement et/ou manuellement, dans lequel
- une surface de section transversale (7) du canal de vis (3) est conçue pour être constante ou augmentant vers la seconde extrémité (6) du canal de vis (3) et la forme résultante est mémorisée en tant que forme finale (8) du canal de vis (3) et
- dans lequel un modèle 3D d'une incrustation (9) avec une surface d'enveloppe (10), une face supérieure (11) et une face inférieure (12) est produit, la surface d'enveloppe (10) étant formée comme une forme négative d'une partie de la forme finale (8) du canal de vis (3), adjacente à la deuxième extrémité (6), **caractérisé en ce qu'**une partie de la surface (2) du modèle 3D de l'élément de prothèse dentaire (1) découpée par la deuxième extrémité (6) de la forme finale (8) du canal de vis (3) est utilisée comme face supérieure (11) et la surface d'enveloppe (10) est conçue comme une forme négative d'une partie de la forme finale (8) du canal de vis (3) adjacente à la seconde extrémité (6).

2. Procédé selon la revendication 1, **caractérisé en ce que** pour concevoir l'augmentation du canal de vis (3) sur la surface (2) du modèle 3D de l'élément de prothèse dentaire (1), une ligne (13) entourant dans la direction circonférentielle la forme initiale cylindrique (4) du canal de vis (3) est manuellement et/ou automatiquement produite, **en ce que** une pente ou une longueur de l'augmentation est spécifiée automatiquement et/ou manuellement et **en ce que** la forme finale (6) du canal de vis (3) est conçue avec la pente spécifiée ou augmentant sur la longueur spécifiée jusqu'à la ligne (13).

3. Procédé selon la revendication 1 ou 2, **caractérisé en ce que** la surface d'enveloppe (10) du modèle 3D de l'incrustation (9) est décalée automatiquement et/ou manuellement au moins partiellement en direction d'un axe longitudinal (14) du modèle 3D de l'incrustation (9).

4. Procédé selon l'une des revendications 1 à 3, **caractérisé en ce que** la surface de section transversale (7) du canal de vis dans la zone de l'augmentation est conçue avec une forme non ambiguë et non symétrique en rotation dans la direction circonférentielle pour une sécurité anti-rotation.

5. Structure à incrustation vestibulaire pour la réparation d'un défaut dentaire, composée d'un élément de prothèse dentaire (1) avec un canal de vis traversant (3) pour une vis de fixation et d'une incrustation (9) avec une face supérieure (11), une face inférieure (12) et une surface d'enveloppe (10), le canal de vis (3) présentant au moins dans une région adjacente à une seconde extrémité incisale ou occlusale (6) du canal de vis (3) vers la zone incisale, occlusale ou buccale, une surface de section transversale constante ou augmentant (7) et la surface d'enveloppe (10) correspondant à une forme négative d'une partie du canal de vis (3) adjacente à la surface incisale, occlusale ou buccale, **caractérisée en ce que** la face supérieure (11) représente une continuation de la surface incisale, occlusale ou buccale dans la zone du canal de vis (3).

6. Structure à incrustation vestibulaire selon la revendication 5, **caractérisée en ce que** le canal de vis (3) dans la zone adjacente à la seconde extrémité (6) a une forme non ambiguë dans la direction circonférentielle, qui assure une orientation non ambiguë dans la direction circonférentielle entre l'élément de prothèse dentaire (1) et l'incrustation (9).
